# EUROPEAN PATENT APPLICATION

(11) **EP 4 667 462 A2**
(43) Date of publication of application: **24.12.2025**
(21) Application number: 25195240.4
(22) Date of filing: 03.03.2017
(51) Int. Cl.: C07D 315/00

(54) **PRODUCTION EQUIPMENT FOR PRODUCTION OF CAPROLACTONE**

(30) Priority: 09.03.2016 GB 201604114
(62) Divisional of application: 17763648.7
(71) Applicant: Ingevity UK Ltd, Warrington Cheshire WA4 6HA (GB)
(72) Inventor: MAYO, William, John, Northwich CW9 6BX (GB); EDWARDSON, Neil, David, Warrington WA5 9UT (GB); ORRELL, Antony, Warrington WA1 4QP (GB); HAZLEHURST, Jeremy, Charles, Altrincham WA15 9HG (GB)
(74) Representative: Forresters IP LLP

(57) **Abstract**

Disclosed is a production equipment for production of a caprolactone by reaction between peracetic acid, produced from acetic acid and hydrogen peroxide, and cyclohexanone, said equipment being split into a first section (Fig. 1) wherein peracetic acid is produced, a second section (Fig. 2) wherein caprolacton is produced, and a third section (Fig. 3) wherein yielded product is purified.

## Description

The present invention refers to a production equipment for production of a caprolactone, such as ε-caprolactone, by reaction between peracetic acid and cyclohexanone in a so called Baeyer-Villiger reaction.

The first step in a caprolactone synthesis is typically the generation of peracetic acid, from acetic acid and hydrogen peroxide, used to oxidise cyclohexanone according to reaction scheme (I) below

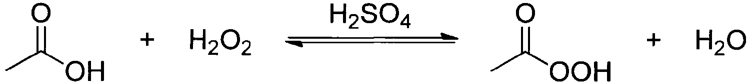

Yielded peracetic acid reacts with cyclohexanone to yield caprolactone and acetic acid according to reaction scheme (II) below

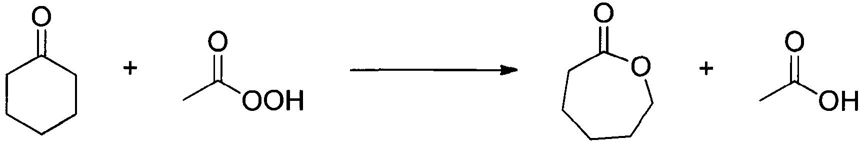

The present invention is directed to a production equipment for production of a caprolactone by above disclosed reactions, said equipment is in embodiments split into a first section wherein peracetic acid is produced, a second section wherein caprolacton is produced, and a third section wherein yielded product is purified.

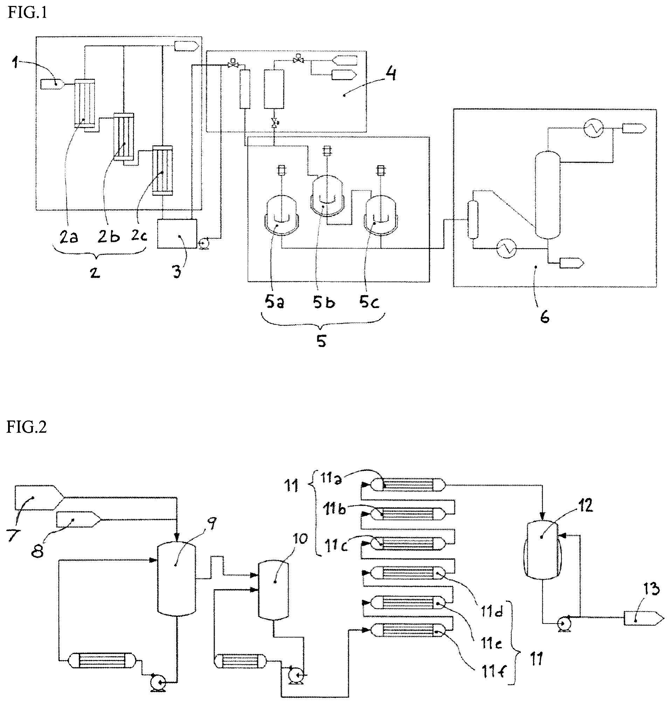

Said first section (Fig. 1) comprises, in embodiments of the present invention, a hydrogen peroxide inlet (1), a concentration unit (2) wherein hydrogen peroxide is concentrated into high strength hydrogen peroxide, a high strength hydrogen peroxide tank (3), a measure unit (4) wherein said high strength hydrogen peroxide and acetic acid are measured before being mixed and cooled in a premix unit (5), and peracetic acid stills (6) wherein said high strength hydrogen peroxide and said acetic acid is reacted in presence of a catalyst, such as sulphuric acid, and fractionated by distilling off yielded peracetic acid, unreacted acetic acid and water and holding back unreacted peroxide and catalyst. Yielded peracetic acid is subsequently and after addition of a stabiliser, such as dipicolinic acid, sent to a storage tank for further feeding to said second section (Fig.2).

Said concentration unit (2) comprises, in especially preferred embodiments of said first section (Fig. 1), a serious of 3 peroxide concentrators (2a, 2b and 2c) being for instance film evaporators having large surface areas. In likewise preferred embodiments said premix unit (5) comprises a series of 3 agitated vessels (5a, 5b and 5c). 60-70%, such as 70%, hydrogen peroxide is in said concentration unit (2), at a pressure of for instance 20-45 mBar and at a temperature of 50-60°C, such as 55 +/-2°C, concentrated into high strength peroxide comprising at least 80%, such as at least 85%, hydrogen peroxide. The peracetic acid stills (6) are in said preferred embodiments suitably operated at 70-150, such as 110-130, mBar and yielded peracetic acid has typically a peracetic acid concentration of 30-55%.

Said second section (Fig. 2) comprises a peracetic inlet (7), a cyclohexanone inlet (8), at least two continuously stirred reactors (9 and 10) followed by a set of tubular reactors (11), in especially preferred embodiments at least 4, such as 6, tubular reactors (11a, 11b, 11c, 11d, 11e and 11f) in series wherein said peracetic acid and said cyclohexanone are reacted, a product cooler (12) wherein yielded reaction mixture is cooled, and an outlet (13) for further feeding of yielded reaction mixture to said third section.

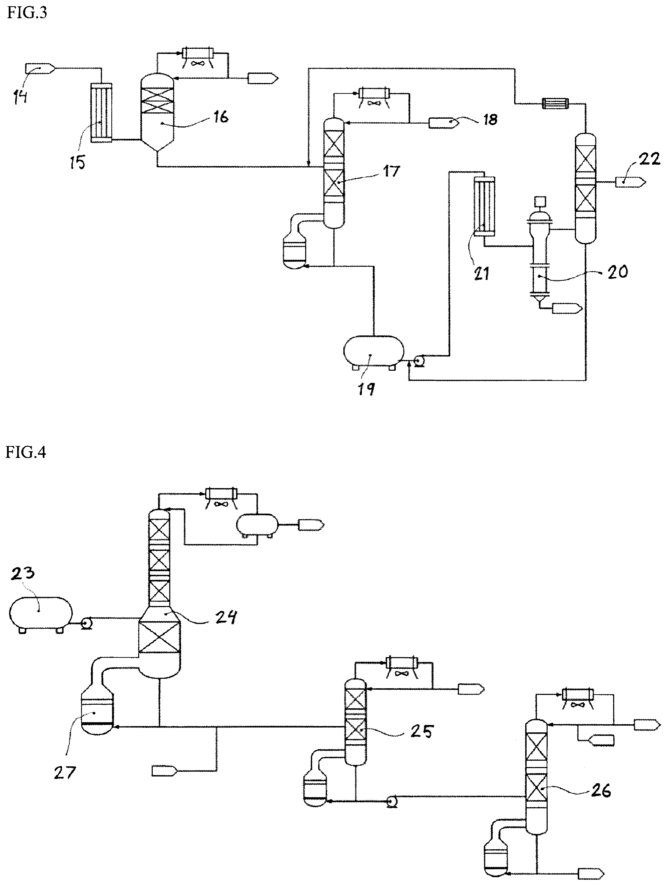

Said third sector (Fig. 3) comprises, in preferred embodiments of the present invention, an inlet (14) for feeding of yielded reaction mixture, a pre-heater (15) heating the reaction mixture to a predetermined temperature, such as 50 +/- 5°C, a stripper (16) wherein water is removed, a fractionator (17) wherein light ends and unreacted raw materials are removed through an outlet (18). The fractionated product is fed to a tank (19) and subsequently to a thin film evaporator (20) via a pre-heater (21) and finally to a distillation column (22), operating at for instance 0.5-20, such as 5-10, mbar, comprising separation steps yielding purified caprolactone.

The production equipment according to the present invention may optionally and additionally comprise a forth section (Fig. 4) comprising a tank (23) comprising unreacted raw materials and light ends which products are separated in at least a primary (24), a secondary (25) and a tertiary (26) distillation column, whereby cyclohexanone and acetic acid are recovered and optionally recycled. Said primary distillation column (24) is, in preferred embodiments, operated at for instance atmospheric pressure, whereby an azeotrope of water and cyclohexanone is distilled off and separated in a decanter (27). Acetic acid is, in said secondary distillation column (25), which suitably operates at for instance a temperature of 110-120°C, distilled off and sent to a storage tank. Cyclohexanone is, under for instance vacuum condition, purified in said tertiary distillation column (26) and remains are sent for disposal.

While particular embodiments of the invention have been shown, it will be understood, of course, that the invention is not limited thereto since many modifications may be made, and it is, therefore, contemplated to cover by the appended claims any such modifications as fall within the true spirit and scope of the invention. These and other objects will be more fully understood from appended drawings, wherein like reference numerals have been applied to like parts throughout the various figures and wherein:
Figur 1: Schemes an embodiment of said first section of the claimed production equipment,
Figur 2: Schemes an embodiment of said second section of the claimed production equipment,
Figur 3: Schemes an embodiment of said third section of the claimed production equipment, and
Figur 4: Schemes an embodiment of said optional forth section of the claimed production equipment.

The present invention will now be described with reference to the following clauses:
1. A production equipment for production of a caprolactone by reaction between peracetic acid, produced from acetic acid and hydrogen peroxide, and cyclohexanone, said equipment being split into a first section wherein peracetic acid is produced, a second section wherein caprolacton is produced, and a third section wherein yielded product is purified, whereby said first section (Fig. 1) comprises a hydrogen peroxide inlet (1), a concentration unit (2) wherein hydrogen peroxide is concentrated into high strength hydrogen peroxide, a high strength hydrogen peroxide tank (3), a measure unit (4) wherein said high strength hydrogen peroxide and acetic acid are measured before being mixed and cooled in a premix unit (5), and peracetic acid stills (6) wherein said high strength hydrogen peroxide and said acetic acid is reacted in presence of sulphuric acid as catalyst and fractionated by distilling off yielded peracetic acid, unreacted acetic acid and water and holding back unreacted peroxide and sulphuric acid, yielded peracetic acid is subsequently and after addition of dipicolinic acid, as stabiliser, sent to a storage tank for further feeding to said second section (Fig.2) comprising a peracetic inlet (7), a cyclohexanone inlet (8), at least two continuously stirred reactors (9, 10) followed by a set of tubular reactors (11) in series wherein said peracetic acid and said cyclohexanone are reacted, a product cooler (12) wherein yielded reaction mixture is cooled, and an outlet (13) for further feeding of yielded reaction mixture to said third section (Fig. 3) comprising an inlet (14) for feeding of yielded reaction mixture, a pre-heater (15), a stripper (16) wherein water is removed, a fractionator (17) wherein light ends and unreacted raw materials are removed through an outlet (18) and said fractionated product being fed to a tank (19), and subsequently to a thin film evaporator (20) via a pre-heater (21) and finally to a distillation column (22) with separation steps yielding purified caprolactone.
2. The production equipment according to clause 1, wherein said concentration unit (2) comprises a serious of 3 peroxide concentrators (2a, 2b and 2c).
3. The production equipment according to clause 2, wherein said peroxide concentrators (2a, 2b and 2c) are film evaporators having large surface areas.
4. The production equipment according to any of the clauses 1-3, wherein said premix unit (5) comprises a series of 3 agitated vessels (5a, 5b and 5c).
5. The production equipment according to anyone of the clauses 1-4, wherein 60-75% hydrogen peroxide is concentrated into high strength peroxide comprising at least 80% hydrogen peroxide.
6. The production equipment according to anyone of the clauses 1-5, wherein 60-75% hydrogen peroxide is concentrated at 20-45 mBar.
7. The production equipment according to anyone of the clauses 1-6, wherein 60-75% hydrogen peroxide is concentrated at 50-60°C.
8. The production equipment according to anyone of the clauses 1-7, wherein 70% hydrogen peroxide is concentrated at a temperature of 55 +/-2C into high strength peroxide comprising at least 85% hydrogen peroxide.
9. The production equipment according to anyone of the clauses 1-8, wherein yielded peracetic acid has a peracetic acid concentration of 30-55%.
10. The production equipment according to anyone of the clauses 1-9, wherein said peracetic acid stills (6) are operated at 70-150 mBar.
11. A production equipment according to anyone of the clauses 1-11 characterised in that said set of tubular reactors (11) comprises 6 tubular reactors (11a, 11b, 11c, 11d, 11e and 11f) in series.
12. The production equipment according to anyone of the clauses 1-11, wherein said pre-heater (15) heats said reaction mixture to 50 +/- 5°C.
13. The production equipment according to anyone of the clauses 1-12, wherein said distillation column (22) operates at a pressure of 0.5-20 mbar.
14. The production equipment according to anyone of the clauses 1-13, wherein said distillation column (22) operates at a pressure of 5-10 mBar.
15. The production equipment according to anyone of the clauses 1-14, wherein it optionally and additionally comprises a forth section (Fig. 4) comprising a tank (23) comprising unreacted raw materials and light ends which products are separated in at least a primary (24), a secondary (25) and a tertiary (26) distillation column, whereby cyclohexanone and acetic acid are recovered and optionally recycled.
16. The production equipment according to clause 15, wherein said primary distillation column (24) operates at atmospheric pressure and wherein an azeotrope of water and cyclohexanone is distilled off and separated in a decanter (27).
17. The production equipment according to clause 15 or 16, wherein said secondary distillation column (25) operates at a temperature of 110-120°C and wherein acetic acid is distilled off and sent to a storage tank.
18. The production equipment according to anyone of the clauses 15-17, wherein said tertiary distillation column (26) purifies cyclohexanone under vacuum condition and wherein remains are sent for disposal.

## Claims

1. A production equipment for production of a caprolactone by reaction between peracetic acid and cyclohexanone, said production equipment comprising
(a) a first section that produces peracetic acid and comprises:
a hydrogen peroxide inlet (1) that provides hydrogen peroxide to a concentration unit (2) configured to concentrate the hydrogen peroxide to at least 80% hydrogen peroxide;
a hydrogen peroxide tank (3) that is in fluid communication with the concentration unit (2) and configured to receive the at least 80% hydrogen peroxide from the concentration unit;
a measure unit (4) adapted to measure and feed (i) said at least 80% hydrogen peroxide from the hydrogen peroxide tank (3) and (ii) acetic acid from an acetic acid inlet, to a premix unit (5) that adds sulfuric acid to the mixture and is configured to mix and cool the mixture;
peracetic acid stills (6) that is in fluid communication with the premix unit (5) and is configured to (i) add dipicolinic acid to the reaction mixture from the premix unit (5) and (ii) fractionate the reaction mixture from the premix unit (5) by distilling off yielded peracetic acid, unreacted acetic acid and water, and holding back unreacted peroxide and sulphuric acid in the peracetic acid stills (6); and
a storage tank that receives the yielded peracetic acid;
(b) a second section that produces caprolactone and comprises:
a peracetic inlet (7) that receives the yielded peracetic acid-dipocolinic acid mixture from the storage tank of the first section and a cyclohexanone inlet (8), each feeding into at least two continuously stirred reactors (9, 10) connected in series, followed by a set of tubular reactors (11) connected in series, wherein said peracetic acid and said cyclohexanone are reacted; and
a product cooler (12) that receives the yielded reaction mixture from the set of tubular reactors (11) and cools the yielded reaction mixture; and
(c) a third section that purifies a yielded product and comprises:
an inlet (14) that feeds the yielded reaction mixture from the product cooler (12) into a pre-heater (15);
a stripper (16) in fluid communication with the pre-heater (15) and that is configured to remove water from the pre-heated yielded reaction mixture and provide the dewatered yielded reaction mixture to a fractionator (17) that removes light ends and unreacted raw materials through an outlet (18) and a fractionated product that is fed to a tank (19); and
a pre-heater that is in fluid communication with the tank (19) and that provides the pre-heated fractionated product to a thin film evaporator (20) and a distillation column (22) that are in fluid communication, in series and configured to yield purified caprolactone.

2. The production equipment according to claim 1, wherein:
the concentration unit (2) comprises three peroxide concentrators (2a, 2b, 2c) connected in series;
the set of tubular reactions (11) comprises six tubular reactors (11a, 11b, 11c, 11d, 11e, 11f) connected in series; and/or
said peroxide concentrators (2a, 2b and 2c) are film evaporators.

3. The production equipment according to claim 1 or 2, wherein:
said premix unit (5) comprises a first agitated vessel (5a), a second agitated vessel (5b), and a third agitated vessel (5c), wherein said premix unit (5) is configured to feed the mixture to the second and third agitated vessels (5b, 5c) connected in series;
the premix unit (5) is configured to add sulfuric acid to each of the agitated vessels (5a, 5b, 5c); and
the peracetic acid stills (6) is in fluid communication with the first agitated vessel (5a) and the third agitated vessel (5c).

4. The production equipment according to anyone one of claims 1-3, wherein:
the concentration unit (2) is adapted to concentrate 60-75% hydrogen peroxide into peroxide comprising at least 80% hydrogen peroxide; and/or
the peracetic acid stills (6) is configured to distil off yielded peracetic acid that has a peracetic acid concentration of 30-55%.

5. The production equipment according to any one of claims 1-4, further comprising a fourth section comprising a tank (23) comprising unreacted raw materials and light ends received from the outlet (18) of the fractionator (17), which are separated in at least a primary distillation column (24), a secondary distillation column (25), and a tertiary distillation column (26) of the fourth section, connected in series, whereby cyclohexanone and acetic acid are recovered and optionally recycled.

6. The production equipment according to claim 5, wherein
(i) said primary distillation column (24) configured to receive the unreacted raw materials and light ends and is configured to operate at atmospheric pressure to distill off an azeotrope of water and cyclohexanone is distilled off, which is separated in a decanter (27); and/or
(ii) said secondary distillation column (25) configured to receive the bottoms from said primary distillation column (24) and configured to operate at a temperature of 110-120°C and distill off acetic acid, which is sent to a storage tank; and/or
(iii) said tertiary distillation column (26) configured to receive the bottoms from said secondary distillation column (25) and configured to purify cyclohexanone under vacuum condition and send remains for disposal.

7. A method of producing caprolactone, the method comprising:
(a) producing peracetic acid by:
concentrating hydrogen peroxide to at least 80% hydrogen peroxide;
mixing and cooling a reaction between the at least 80% hydrogen peroxide and acetic acid, catalysed with sulfuric acid, in a premix unit (5) to produce a reaction mixture;
fractionating the reaction mixture by distilling off peracetic acid, unreacted acetic acid, and water, wherein unreacted peroxide and sulfuric acid is held back; and
adding dipicolinic acid to the peracetic acid;
(b) producing caprolactone by:
reacting the peracetic acid-dipicolinic acid mixture and cyclohexanone whiles passing through at least two continuous stir reactors (9, 10), followed by a set of tubular reactors (11) to produce a reaction mixture; and
cooling the reaction mixture; and
(c) purifying the reaction mixture by:
preheating the reaction mixture;
removing water from the preheated reaction mixture via a stripper (16);
removing light ends and unreacted materials from the reaction mixture with a fractionator (17) to produce a fractionated product;
preheating the fractionated product; and
separating the preheated fractionated product with a distillation column (22) to produce purified caprolactone.

8. The method of claim 7, wherein:
concentrating hydrogen peroxide is performed with a concentration unit (2) comprising three peroxide concentrators (2a, 2b, and 2c) connected in series;
the set of tubular reactors (11) comprises six tubular reactors (11a, 11b, 11c, 11d, 11e, and 11f) connected in series, to produce a reaction mixture; and/or the peroxide concentrators are film evaporators.

9. The method of claim 7 or 8, wherein:
the premix unit (5) comprises a first agitated vessel (5a), a second agitated vessel (5b), and a third agitated vessel (5c), wherein the mixture is fed to the second and third agitated vessels (5b, 5c) connected in series;
the premix unit (5) adds sulfuric acid to each of the agitated vessels (5a, 5b, 5c); and
fractionating is performed on streams received from the first agitated vessel (5a) and the third agitated vessel (5c).

10. The method of any one of claims 7-9, wherein concentrating hydrogen peroxide includes
concentrating a 60-75% hydrogen peroxide feedstock to the at least 80% hydrogen peroxide,
concentrating a 60-75% hydrogen peroxide at 20 to 45 mBar and/or at a temperature of 50 to 60 °C, and/or
concentrating 70% hydrogen peroxide at a temperature of 55 +/-2 °C into peroxide comprising at least 85% hydrogen peroxide.

11. The method of any one of claims 7-10, wherein the peracetic acid has a peracetic acid concentration of 30% to 55%.

12. The method of any one of claims 7-11, wherein
fractionating the reaction mixture is performed at 70 to 150 mBar;
preheating the reaction mixture includes heating the reaction mixture to 50 +/- 5 °C; and/or
distilling the fractionated product is performed in a distillation column (22) that is operated at a pressure of 0.5 to 20 mbar.

13. The method of claim 12, wherein distilling the fractionated product is performed in the distillation column (22) that is operated at a pressure of 5 to 10 mBar.

14. The method of anyone of claims 7-13, further comprising (d) recovering unreacted acetic acid and cyclohexanone by separating the light ends from (i) distilling off unreacted acetic acid and (ii) the distillation column (22) in at least a primary distillation column (24), a secondary distillation column (25), and a tertiary distillation column (26), optionally further comprising recycling the recovered cyclohexanone and acetic acid.

15. The method of claim 14, wherein separating the light ends comprises:
(i) distilling off azeotrope of water and cyclohexanone in the primary distillation column (24), operated at atmospheric pressure and separating the azeotrope of water and cyclohexanone in a decanter (27);
(ii) distilling off acetic acid from the bottoms from the primary distillation column (24) in the secondary distillation column (25) operated at a temperature of 110-120 °C; and/or
(iii) purifying the cyclohexanone from the bottoms of the second distillation column (25) in the tertiary distillation column (26) while under vacuum condition, wherein remains are sent for disposal.
